(19) Europäisches Patentamt
European Patent Office
Office européen des brevets

(11) Veröffentlichungsnummer: **0 029 964**
**A1**

(12) # EUROPÄISCHE PATENTANMELDUNG

(21) Anmeldenummer: **80107210.9**

(22) Anmeldetag: **20.11.80**

(51) Int. Cl.³: **C 07 C 93/14**
C 07 D 295/08, A 61 K 7/13

(30) Priorität: **29.11.79 DE 2948093**

(43) Veröffentlichungstag der Anmeldung:
**10.06.81 Patentblatt 81/23**

(84) Benannte Vertragsstaaten:
**AT BE CH DE FR GB IT LI NL SE**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Rose, David, Dr.**
**Holbeinweg 7**
**D-4010 Hilden(DE)**

(72) Erfinder: **Lieske, Edgar**
**Hunsrückenstrasse 40**
**D-4000 Düsseldorf-1(DE)**

(54) **Neue Kupplerkomponenten für Oxidationshaarfarben, deren Herstellung und Verwendung sowie diese enthaltende Haarfärbemittel.**

(57) Durch einen der Reste $-NHC_2H_5$, $-NHC_6H_5$, $-NHCH_2C_6H_5$, $-N(C_2H_5)(C_6H_5)$, -OH, $-OC_2H_5$, Morpholinrest oder Piperidinrest in 1-Stellung substituierte 2-Hydroxypropyl-(2, 4-diaminophenolether, deren Herstellung und Verwendung als Kupplerkomponenten in Oxidationshaarfarben, sowie diese enthaltende Haarfärbemittel.

EP 0 029 964 A1

HENKEL KGaA
ZR-FE/Patente
z-sü

# P a t e n t a n m e l d u n g

D 6083 EP

"Neue Kupplerkomponenten für Oxidationshaarfarben, deren Herstellung und Verwendung sowie diese enthaltende Haarfärbemittel"

---

Gegenstand der Erfindung sind neue 1-substituierte 2-Hydroxypropyl-(2,4-diaminophenolether) der allgemeinen Formel

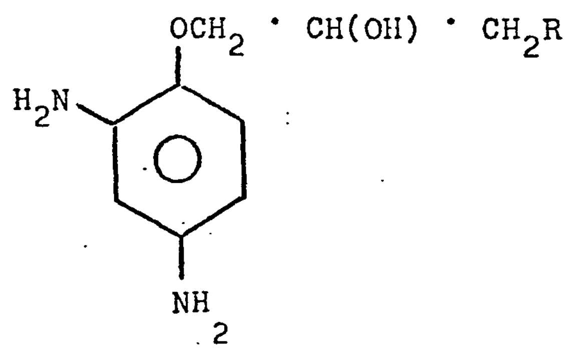

in der R einen der Reste $-NHC_2H_5$, $-NHC_6H_5$, $-NHCH_2C_6H_5$, $-N(C_2H_5)(C_6H_5)$, $-OH$, $-OC_2H_5$, Morpholin- oder Piperidinrest darstellt.

Die Herstellung der neuen 1-substituierten 2-Hydroxypropyl-(2,4-diaminophenolether) erfolgt nach bekannten Verfahren der organischen Chemie in mehreren Stufen. Zunächst wird in erster Stufe 2-Acetamido-4-nitrophenol mit Epichlorhydrin zum 1-(2-Acetamido-4-nitrophenoxy)-2,3-epoxypropan umgesetzt. Hieran wird in zeiter Stufe durch Umsetzung mit einem Amin, Wasser oder Alkohol unter Öffnung des Oxiranringes und Ausbildung einer Hydroxylgruppe in 2-Stellung des Propylrestes in 3-Stellung des Propylrestes der gewünschte Rest R eingeführt. In dritter Stufe wird hernach der zur Maskierung der Aminogruppe dienende Acetylrest abgespalten, wonach in 4. Stufe durch katalytische Hydrierung die Nitrogruppe in die Aminogruppe überführt und somit das gewünschte neue

/2

Produkt erhalten wird. Die Reaktion verläuft dabei nach folgendem Schema, wobei R die vorgenannte Bedeutung hat:

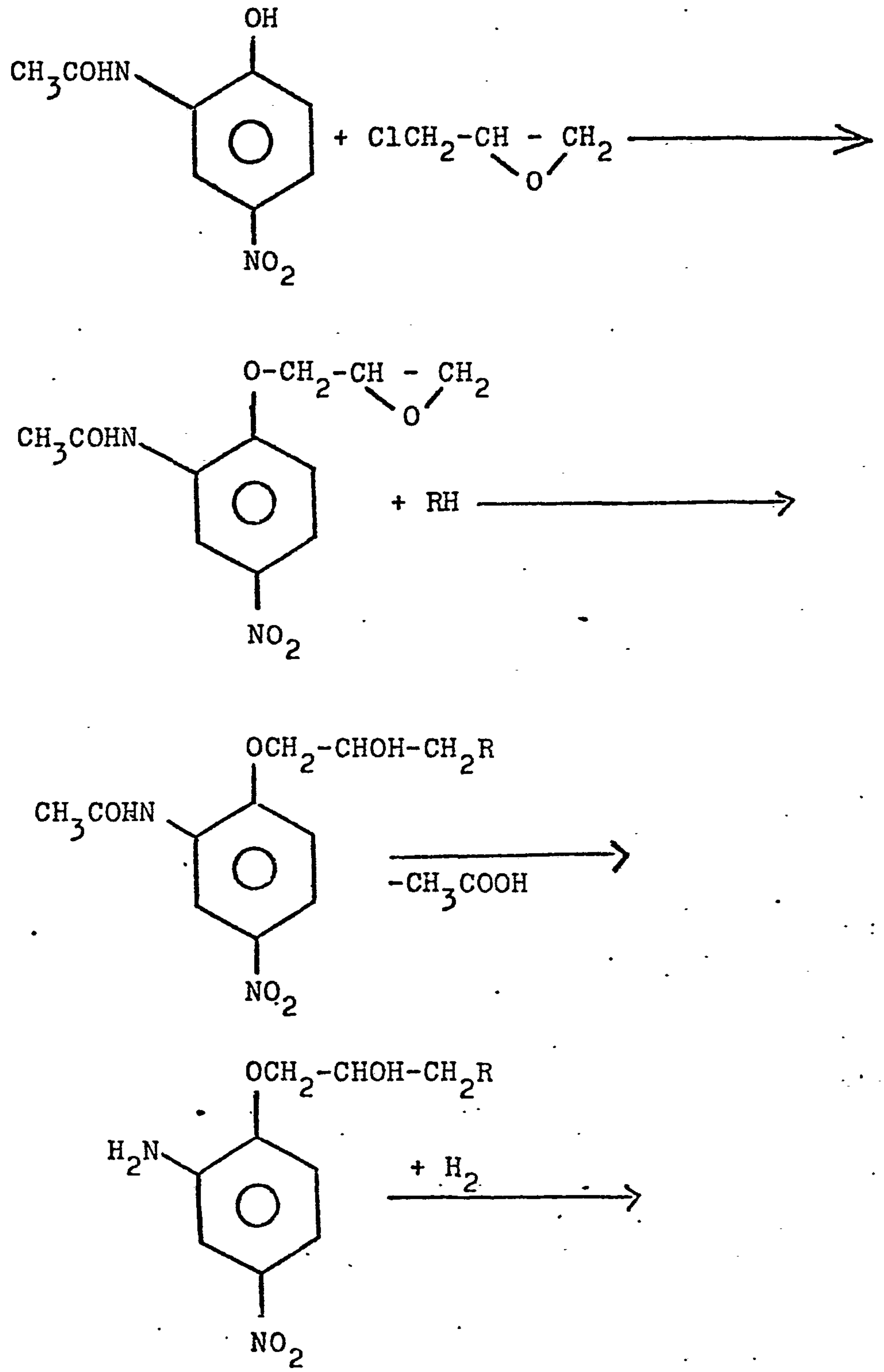

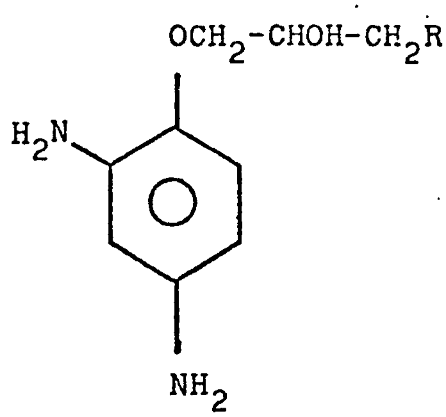

Weitere Gegenstände der Erfindung sind die Verwendung der neuen 1-substituierten 2-Hydroxypropyl-(2,4-diaminophenolether) als solcher oder in Gestalt ihrer Salze mit anorganischen oder organischen Säuren als Kupplerkomponenten in Oxidationshaarfarben sowie Haarfärbemittel, die die neuen 1-substituierten 2-Hydroxypropyl-(2,4-diaminophenolether) bzw. deren Salze enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolon-derivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen:

Sie müssem bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes

Aufziehvermögen auf menschlichem Haar besitzen und sie sollen darüber hinaus in toxikologischer und dermatologischer Hinsicht unbedenklich sein.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, gelangt, wenn man als Kupplerkomponenten 1-substituierte 2-Hydroxypropyl-(2,4-diaminophenolether) der allgemeinen Formel

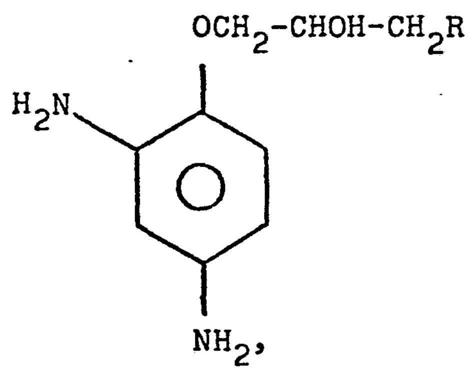

in der R einen der Reste $-NHC_2H_5$, $-NHC_6H_5$, $-NHCH_2C_6H_5$, $-N(C_2H_5)(C_6H_5)$, $-OH$, $-OC_2H_5$, Morpholin- oder Piperidinrest darstellt, sowie deren Salze mit anorganischen oder organischen Säuren in Kombination mit üblichen Entwicklersubstanzen verwendet.

Haarfärbemittel auf Basis von Oxidationsfarben mit einem Gehalt an 1-substituierten 2-Hydroxypropyl-(2,4-diaminophenolethern) der allgemeinen Formel

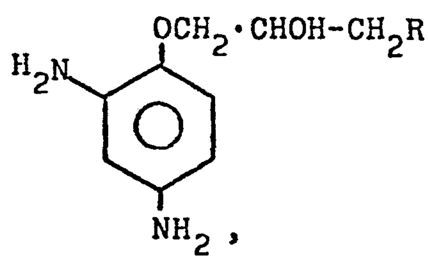

in der R die vorgenannte Bedeutung hat, sowie deren Salzen mit anorganischen oder organischen Säuren als Kupplerkomponenten und den in Oxidationshaarfarben üblichen Entwicklersubstanzen stellen demnach besonders wertvolle Kompositionen auf dem Gebiet der Oxidationshaarfarben dar.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive, von türkis bis dunkelblau reichende Farbtöne und stellen somit eine wesentliche Bereicherung der oxidativen Haarfärbemöglichkeiten dar. Darüber hinaus zeichnen sich die erfindungsgemäßen 1-substituierten 2-Hydroxypropyl-(2,4-diaminophenolether) durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Kupplerkomponenten zu verwendenden 1-substituierten 2-Hydroxypropyl-(2,4-diaminophenolether) können entweder als solche oder in Form ihrer Salze mit anorganischen oder organischen Säuren, wie zum Beispiel als Chloride, Sulfate, Phosphate, Acetate, Propionate, Lactate, Citrate eingesetzt werden.

Als erfindungsgemäß einzusetzende Kupplerkomponenten sind 1-(2,4-Diaminophenoxy)-3-anilino-propanol-2, 1-(2,4-Diaminophenoxy)-3-benzylamino-propanol-2, 1-(2,4-Diaminophenoxy)-3-(N-ethylanilino)-propanol-2, 1-(2,4-Diaminophenoxy)-3-ethoxypropanol-2, 1-(2,4-Diaminophenoxy)-3-ethylamino-propanol-2, 1-(2,4-Diaminophenoxy)-3-morpholino-propanol-2, 1-(2,4-Diaminophenoxy)-2,3-propandiol, 1-(2,4-Diaminophenoxy)-3-piperidino-propanol-2 sowie deren Salze mit anorganischen oder organischen Säuren, zu nennen.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, n-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlor-p-phenylendiamin, N,N-Bis-hydroxyethylamino-p-phenylendiamin, Methoxyp-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1 - 4 Kohlenstoffatomen darstellt, ferner Diaminopyridinderivate, heterocyclische Hydrazonderivate wie 1-Methylpyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methylaminopyrimidin, 2,4,5-Triamino-6-dimethylaminopyrimidin, 2,4,5-Triamino-6-piperidino-pyrimidin, 2,4,5-Triamino-6-anilino-pyrimidin, 2,4,5-Triamino-6-morpholino-pyrimidin, 2,4,5-Triamino-6-β-hydroxy-ethylamino-pyrimidin anzuführen.

Die erfindungsgemäßen Kupplerkomponenten liefern neben anderen Farbnuancen mit entsprechenden Entwicklersubstanzen auch dunkelblaue bis schwarzblaue, besonders intensive Haarfärbungen, die sich durch außerordentliche Lichtechtheiten auszeichnen. Sie sind daher auch als Nuancierkomponente zur Erzielung möglichst kräftiger und den natürlichen Haarfarbennuancen weitgehend entsprechender Farbtöne von besonderer Wichtigkeit,

/7

da sich bei der Erstellung natürlicher Farbnuancen unter Zuhilfenahme von Blaukupplerkomponenten häufig Schwierigkeiten ergeben.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden 1-substituierten 2-Hydroxypropyl-(2,4-diaminophenolether) darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 - 10 erfolgen. Die Anwendungstemperaturen bewegen sich dabei im Bereich von 15 bis $40^{o}$ C. Nach einer Einwirkungsdauer von circa

30 Minuten wird das Haarfärbemittel vom zu färbenden Haar durch Spülen entfernt. Hernach wird das Haar mit einem milden Shampoo nachgewaschen und getrocknet.

Die mit den erfindungsgemäßen Haarfärbemitteln erzielbaren Färbungen, insbesondere auch die Blautöne zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

/10

## B e i s p i e l e

Zunächst wird nachstehend die Herstellung der erfindungsgemäßen 1-substituierten 2-Hydroxypropyl-(2,4-diaminophenolether) beschrieben.

A) 1-(2,4-Diaminophenoxy)-3-ethylamino-propanol-2-trihydrochlorid

1. Stufe: Herstellung von 1-(2-Acetamido-4-nitrophenoxy)-2,3-epoxypropan.
147,5 g 2-Acetamido-4-nitrophenol wurden mit 600 ml Epichlorhydrin und 750 ml 1,1 n-Natronlauge 48 Stunden lang bei ca. 20° C gerührt. Die Lösung wurde filtriert und einige Stunden lang bei ca. 20° C gerührt. Das abgeschiedene Produkt wurde abgesaugt und im Vakuum getrocknet. Das erhaltene 1-)2-Acetamido-4-nitrophenoxy)-2,3-epoxypropan schmilzt bei 141 - 143°C.

2. Stufe: Herstellung von 1-(2-Acetamido-4-nitrophenoxy)-3-ethylamino-propanol-2.
8 g (0,03 Mol) 1-(2-Acetamido-4-nitrophenoxy)-2,3-epoxypropan wurden mit 85 g (0,94 Mol) Ethylamin (50 %ige wäßrige Lösung) 5 Stunden lang bei ca. 20° C gerührt. Das ausgefallene Produkt wurde abgesaugt und mit Wasser nachgewaschen. Nach dem Trocknen erhielt man 1-(2-Acetamido-4-nitrophenoxy)-3-ethylamino-propanol-2 mit einem Schmelzpunkt von 168 - 170° C.

/11

3. Stufe: Herstellung von 1-(2-Amino-4-nitrophenoxy)-3-ethylaminopropanol-2-hydrochlorid.
6,1 g 1-(2-Acetamido-4-nitrophenoxy)-2,3-propanol-2 wurden mit 80 ml 2n-Salzsäure während 2,5 Stunden unter Rückfluß gekocht. Die Lösung wurde zur Trockne eingeengt. Das erhaltene 1-(2-Amino-4-nitrophenoxy)-3-ethylaminopropanol-2-hydrochlorid schmilzt bei 90 - 95° C.

4. Stufe: Herstellung von 1-(2,4-Diaminophenoxy)-3-ethylamino-propanol-2-trihydrochlorid.
6,1 g 1-(2-Amino-4-nitrophenoxy)-3-ethylaminopropanol-2-hydrochlorid wurden in 50 ml Ethanol in Gegenwart von 5 % Palladium auf Kohle katalytisch hydriert. Nach beendeter Wasserstoffaufnahme wurde vom Katalysator abgetrennt. Die Lösung wurde mit konzentrierter Salzsäure angesäuert und zur Trockne eingeengt. Die gewünschte Verbindung 1-(2,4-Diaminophenoxy)-3-ethylamino-propanol-2-trihydrochlorid wurde in Gestalt weißer Kristalle erhalten, die bei 95° C unter Zersetzung schmelzen.

B) 1-(2,4-Diaminophenoxy)-3-piperidino-propanol-2-trihydrochlorid

1. Stufe: In erster Stufe wurde entsprechend der ersten Stufe von A) 1-(2-Acetamido-4-nitrophenoxy)-2,3-epoxypropan hergestellt.

2. Stufe: Herstellung von 1-(2-Acetamido-4-nitrophenoxy)-3-piperidino-propanol-2.
8 g (0,03 Mol) 1-(2-Acetamido-4-nitrophenoxy)-2,3-epoxypropan wurden mit 38,3 g (0,45 Mol)

Piperidin entsprechend den Angaben der zweiten Stufe von A) umgesetzt. Das erhaltene gelbe Öl wurde in dritter Sufe weiterverarbeitet.

3. Stufe: Herstellung von 1-(2-Amino-4-nitrophenoxy)-3-piperidino-propanol-2-hydrochlorid. Die Abspaltung des Acetylrestes wurde analog Stufe 3 von A durchgeführt. Die Verbindung 1-(2-Amino-4-nitrophenoxy)-3-piperidino-propanol-2-hydrochlorid wurde in Form von gelben Kristallen erhalten, die bei 180° C unter Zersetzung schmelzen.

4. Stufe: Herstellung von 1-(2,4-Daiminophenoxy)-3-piperidino-propanol-2-trihydrochlorid. Die Hydrierung erfolgte analog den Angaben der Stufe 4 von A) und ergab die gewünschte Verbindung in Gestalt weißer Kristalle vom Schmelzpunkt 104 - 108° C.

C) 1-(2,4-Diaminophenoxy)-3-morpholino-propanol-2-trihydrochlorid

1. Stufe: Es wurde wiederum 1-(2-Acetamido-4-nitrophenoxy)-2,3-epoxypropan entsprechend A) für die Weiterverarbeitung hergestellt.

2. Stufe: Herstellung von 1-(2-Acetamido-4-nitrophenoxy)-3-morpholino-propanol-2. Die Herstellung erfolgte analog Stufe 2 von A) aus 8 g (0,03 Mol) 1-(2-Acetamido-4-nitrophenoxy)-2,3-epoxypropan und 40 (0,45 Mol) Morpholin und führte zu der Verbindung in Form weißer Kristalle vom Schmelzpunkt 77 - 80° C.

3. Stufe: Herstellung von 1-(2-Amino-4-nitrophenoxy)-3-morpholino-propanol-2-hydrochlorid. Durch Abspaltung des Acetylrestes entsprechend den Angaben in A) wurde das gewünschte Produkt in Form gelber Kristalle erhalten.

4. Stufe: Herstellung von 1-(2,4-Diaminophenoxy)-3-morpholino-propanol-2-trihydrochlorid. Das in 3. Stufe erhaltene Produkt wurde entsprechend den Angaben bei A) hydriert und lieferte die gewünschte Verbindung in Form weißer Kristalle vom Schmelzpunkt 80° C.

D) 1-(2,4-Diaminophenoxy)-3-anilino-propanol-2-trihydrochlorid

1. Stufe: Herstellung von 1-(2-Acetamido-4-nitrophenoxy)-2,3-epoxypropan gemäß A).

2. Stufe: Herstellung von 1-(2-Acetamido-4-nitrophenoxy)-3-anilino-propanol-2 gemäß A) aus 8 g (0,03 Mol) 1-(2-Acetamido-4-nitrophenoxy)-2,3-epoxypropan und 40 g (0,45 Mol) Anilin. Das Produkt wurde in Form weißer Kristalle vom Schmelzpunkt 149 - 152° C erhalten.

3. Stufe: Herstellung von 1-(2-Amino-4-nitrophenoxy)-3-anilino-propanol-2-hydrochlorid. Das Produkt wurde entsprechend den Angaben der 3. Stufe von A in Gestalt gelber Kristalle vom Schmelzpunkt 115 - 120° C erhalten.

4. Stufe: Herstellung von 1-(2,4-Diaminophenoxy)-3-anilino-propanol-2-trihydrochochlorid. Bei der Hydrierung des in 3. Stufe erhaltenen Produktes analog den Angaben bei A) wurde die gewünschte Verbindung als weiße Kristalle erhalten, die bei 116° C unter Zersetzung schmelzen.

E) 1-(2,4-Diaminophenoxy)-3-(N-Ethylanilino)-propanol-2-trihydrochlorid

1. Stufe: Herstellung von 1-(2-Acetamido-4-nitrophenoxy)-2,3-epoxypropan gemäß A).

2. Stufe: Die Herstellung von 1-(2-Acetamido-4-nitrophenoxy)-3-(N-ethylanilino)-propanol-2 erfolgte gemäß den Angaben bei A) aus 8 g (0,03 Mol) 1-(2-Acetamido-4-nitrophenoxy)-2,3-epoxypropan und 54,5 g (0,er Mol) N-Ethylanilin während 5 Stunden bei 60° C und lieferte die Verbindung in Form oranger Kristalle vom Schmelzpunkt 80 - 84° C.

3. Stufe: Die Abspaltung des Acetylrestes entsprechend den Angaben der 3. Stufe von A) ergab 1-(2-Amino-4-nitrophenoxy)-3-(N-ethylanilino)-propanol-2-hydrochlorid als hygroskopische gelbe Kristalle vom Schmelzpunkt 125 - 130° C.

4. Stufe: Herstellung von 1-(2,4-Diaminophenoxy)-3-(N-ethylanilino)-propanol-2-trihydrochlorid.

Durch Hydrierung des in 3. Stufe erhaltenen Produktes wurde die gewünschte Verbindung in Gestalt hygroskopischer weißer Kristalle vom Schmelzpunkt 80 - 85° C erhalten.

F) <u>1-(2,4-Diaminophenoxy)-3-benzylamino-propanol-2-trihydrochlorid</u>

<u>1. Stufe:</u> Herstellung von 1-(2-Acetamido-4-nitrophenoxy)-2,3-epoxypropan gemäß A).

<u>2. Stufe:</u> Entsprechend den Angaben bei A) wurde 1-(2-Acetamido-4-nitrophenoxy)-3-benzylamino-propanol-2 aus 8 g (0,03 Mol) 1-(2-Acetamido-4-nitrophenoxy)-2,3-epoxypropan durch Umsetzung mit 48 g (0,45 Mol) Benzylamin in Form gelboranger Kristalle vom Schmelzpunkt 73 - 77° C erhalten.

<u>3. Stufe:</u> Die Abspaltung des Acetylrestes führte zu 1-(2-Amino-4-nitrophenoxy)-3-benzylamino-propanol-2-hydrochlorid in Gestalt hellgelber Kristalle vom Schmelzpunkt 108 - 112°C.

<u>4. Stufe:</u> Durch Hydrierung des in der 3. Stufe erhaltenen Produktes wurde die gewünschte Verbindung 1-(2,4-Diaminophenoxy)-3-benzylaminopropanol-2-trihydrochlorid in Form hygroskopischer hellvioletter Kristalle erhalten.

G) <u>1-(2,4-Diaminophenoxy)-3-ethoxy-propanol-2-dihydrochlorid</u>

<u>1. Stufe:</u> Herstellung von 1-(2-Acetamido-4-nitrophenoxy)-2,3-epoxypropan gemäß A).

2. Stufe: Analog der Herstellung bei A) wurde 1-(2-Acetamido-4-nitrophenoxy)-3-ethoxypropanol-2 durch Umsetzung von 8 g (0,05 Mol) 1-(2-Acetamido-4-nitrophenoxy)-2,3-epoxypropan mit 50 ml Ethanol in Gegenwart von 0,2 ml conc. Schwefelsäure unter 8-stündigem Kochen unter Rückfluß gewonnen. Die Verbindung wurde nach Einengen als gelbes Öl erhalten.

3. Stufe: Entsprechend den Herstellungsangaben bei A) wurde 1-(2-Amino-4-nitrophenoxy)-3-ethoxypropanol-2-hydrochlorid in Form olivgrüner Kristalle vom Schmelzpunkt 75° C erhalten.

4. Stufe: Die Hydrierung entsprechend A) lieferte das gewünschte 1-(2,4-Diaminophenoxy)-3-ethoxypropanol-2-dihydrochlorid als beige Kristalle, die unter Zersetzung bei 93° C schmelzen.

H) 1-(2,4-Diaminophenoxy)-propandiol-2,3-dihydrochlorid

1. Stufe: Herstellung von 1-(2-Acetamido-4-nitrophenoxy)-2,3-epoxypropan gemäß A).

2. Stufe: Die Herstellung von 1-(2-Acetamido-4-nitrophenoxy)-2,3-propandiol erfolgte gemäß A) durch Kochen von 5 g (0,019 Mol) 1-(2-Acetamido-4-nitrophenoxy)-2,3-epoxypropan mit 50 ml Wasser in Gegenwart von 0,05 g conc. Schwefelsäure unter Rückfluß während 8 Stunden. Das erhaltene schmierige Produkt wurde abgesaugt und in Stufe 3 eingesetzt.

<u>3. Stufe:</u> Die Abspaltung des Acetylrestes gemäß den Angaben bei A ergab 1-(2-Amino-4-nitrophenoxy)-2,3-propandiolhydrochlorid in Form gelber Kristalle vom Schmelzpunkt 152 - 157° C.

<u>4. Stufe:</u> Durch Hydrierung des Produktes der 3. Stufe wurde 1-(2,4-Diaminophenoxy)-2,3-propandioldihydrochlorid in Gestalt weißer Kristalle vom Schmelzpunkt 95° C erhalten.

Die vorgenannten, in ihrer Herstellung beschriebenen 1-substituierten 2-Hydroxypropyl-(2,4-diaminophenolether) A) bis H) wurden als Kupplerkomponenten in den folgenden Beispielen eingesetzt.

Als Entwicklerkomponenten dienten folgende Substanzen:

E 1: p-Toluylendiamin
E 2: 2,4,5,6-Tetraaminopyrimidin
E 3: 2,5-Diaminoanisol
E 4: N,N-Bis-(β-hydroxyethyl)-p-phenylendiamin
E 5: 2-Methylamino-4,5,6-triaminopyrimidin
E 6: 2-Chlor-p-phenylendiamin
E 7: 1-Phenyl-3-carbamoyl-4-amino-pyrazolon-5
E 8: p-Aminophenol
E 9: N,N-Dimethyl-p-phenylendiamin
E10: N-Ethyl-N'-β-hydroxyethyl-p-phenylendiamin.

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}$-$C_{18}$,
10 Gew.-Teilen Fettalkoholsulfat (Natriumsalz) der Kettenlänge $C_{12}$-$C_{18}$ und
75 Gew.-Teilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 % iger Wasserstoffperoxidlösung als Oxidationsmittel durch geführt, wobei zu 100 Gewichtsteilen der Emulsion 10 Gewichtsteile Wasserstoffperoxidlösung gegeben wurden. Die jeweilige Färbecreme mit Zusatz von Oxidationsmitteln wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

/19

Tabelle 1

| Beispiel | Entwickler | Kuppler | Erhaltener Farbton mit 1 %iger $H_2O_2$-Lösung |
|---|---|---|---|
| 1 | E 1 | A | dunkelblau |
| 2 | E 2 | A | grün |
| 3 | E 1 | B | dunkelblau |
| 4 | E 2 | B | grüngrau |
| 5 | E 1 | C | blau |
| 6 | E 2 | C | oliv |
| 7 | E 3 | D | dunkelblau |
| 8 | E 4 | D | dunkelblau |
| 9 | E 5 | D | dunkelgrün |
| 10 | E 6 | D | dunkelviolett |
| 11 | E 7 | D | violettgrau |
| 12 | E 8 | D | rotbraun |
| 13 | E 9 | D | schwarzblau |
| 14 | E10 | D | dunkelblau |
| 15 | E 1 | E | blaugrau |
| 16 | E 2 | E | grautürkis |
| 17 | E 1 | F | blaugrau |
| 18 | E 2 | F | dunkelgrün |
| 19 | E 1 | G | blaugrau |
| 20 | E 2 | G | mattgrün |
| 21 | E 1 | H | dunkelblau |
| 22 | E 2 | H | grün |

"Neue Kupplerkomponenten für Oxidationshaarfarben, deren Herstellung und Verwendung sowie diese enthaltende Haarfärbemittel"

## Patentansprüche:

1. 1-Substituierte 2-Hydroxypropyl-(2,4-diaminophenolether) der allgemeinen Formel

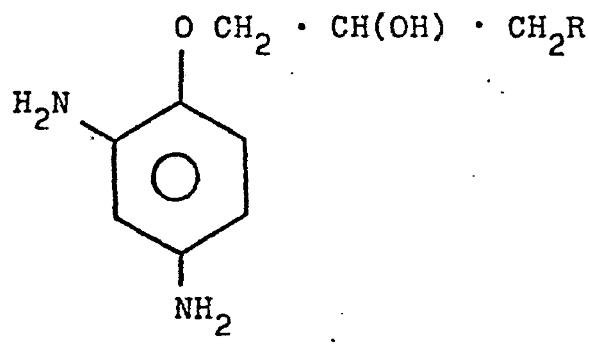

in der R einen der Reste $-NHC_2H_5$, $-NHC_6H_5$, $-NHCH_2C_6H_5$, $-N(C_2H_5)(C_6H_5)$, $-OH$, $-OC_2H_5$, Morpholinrest oder Piperidinrest darstellt.

2. Verfahren zur Herstellung der 1-substituierten 2-Hydroxypropyl-(2,4-diaminophenolether) gemäß Anspruch 1 dadurch gekennzeichnet, daß man in erster Stufe 2-Acetamido-4-nitrophenol mit Epichlorhydrin zum 1-(2-Acetamido-4-nitrophenoxy)-2,3-epoxypropan umsetzt, hieran in zweiter Stufe durch Umsetzung mit einem Amin, Wasser oder Alkohol unter Öffnung des Oxiranringes und Ausbildung einer Hydroxylgruppe in 2-Stellung des Propylrestes, in 3-Stellung des Propylrestes den gewünschten Rest R einführt, danach in dritter Stufe den Acetylrest abspaltet und zuletzt in vierter Stufe durch katalytische Hydrierung die Nitrogruppe in die Aminogruppe überführt.

3. Verwendung von 1-substituierten 2-Hydroxypropyl-(2,4-diaminophenolethern) nach Anspruch 1 und 2, sowie deren Salzen mit anorganischen oder organischen Säuren als Kupplerkomponenten in Oxidationshaarfarbstoffen.

4. Haarfärbemittel auf Basis von Oxidationsfarbstoffen mit einem Gehalt an 1-substituierten 2-Hydroxypropyl-(2,4-diaminophenolethern) nach Anspruch 1 - 3, sowie deren Salzen mit anorganischen oder organischen Säuren als Kupplerkomponenten und den in Oxidationshaarfarben üblichen Entwicklersubstanzen.

5. Haarfärbemittel nach Anspruch 4, gekennzeichnet durch einen Gehalt an Entwickler-Kuppler-Kombination in einer Menge von 0,2 bis 5 Gewichtsprozent, vorzugsweise 1 bis 3 Gewichtsprozent, bezogen auf das gesamte Haarfärbemittel.

0029964

Europäisches Patentamt

# EUROPÄISCHER RECHERCHENBERICHT

Nummer der Anmeldung
EP 80107210.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 737 138 (L'OREAL)<br>+ Patentansprüche 1,6,37 +<br>-- | 1,3-5 | C 07 C 93/14<br>C 07 D 295/08<br>A 61 K 7/13 |
| | DE - A1 - 2 758 735 (BRISTOL-MEYERS)<br>+ Patentansprüche 1,4 +<br>---- | 1,3-5 | |

RECHERCHIERTE SACHGEBIETE (Int Cl.³)

C 07 C 93/00
A 61 K 7/00
C 07 D 295/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 10-02-1981 | REIF |